# EUROPEAN PATENT APPLICATION

(11) **EP 3 508 584 A1**
(43) Date of publication of application: **10.07.2019**
(21) Application number: 19156865.8
(22) Date of filing: 28.01.2014
(51) Int. Cl.: C12P 19/56

(54) **METHOD FOR PREPARING REBAUDIOSIDE M BY USING ENZYME METHOD**

(62) Divisional of application: 14880989.0
(71) Applicant: PepsiCo, Inc., Purchase, New York 10577 (US)
(72) Inventor: TAO, Junhua, Zhangjiagang Jiangsu 215600 (CN); LI, Guoqing, Zhangjiagang Jiangsu 215600 (CN); LIANG, Xiaoliang, Zhangjiagang Jiangsu 215600 (CN); HOU, Maoqi, Zhangjiagang Jiangsu 215600 (CN); TAO, Andrew, Zhangjiagang Jiangsu 215600 (CN)
(74) Representative: Jenkins, Peter David

(57) **Abstract**

Provided is a method for preparing Rebaudioside M by using an enzyme method. In the method, Rebaudioside A or Rebaudioside D is used as a substrate, and in the presence of sucrose and UDP, Rebaudioside M is generated by means of reaction of the substrate under the catalysis of a mixture of UDP-glucosyl transferase and sucrose synthetase, or recombinant cells containing the UDP-glucosyl transferase and sucrose synthetase. The reaction is carried out in an aqueous-phase system at a temperature of 20 to 60°C and pH 5.0 to 9.0. The reaction system further contains dimethyl sulfoxide at a concentration of 3% to 5% according to the ratio by volume for facilitating solubilization of the substrate.

## Description

### Technical Field

The present invention relates to a method for preparing Rebaudioside M, and in particular to a biological method for preparing Rebaudioside M.

### Background

Sugar substitutes can be divided into 4 classes with different influences on human health. The first class includes natural sweeteners such as fruit juices, honey and maple syrup, which are similar to sugar in terms of calorie and carbohydrate content. The second class includes artificial sweeteners, such as aspartame and saccharin that provide no calories and are thus considered as non-nutritive sweeteners. The third class includes sugar alcohols, such as xylitol and sorbitol, mainly derived from vegetables and fruits. The fourth class includes sweet substances extracted from natural plants, such as stevioside extracted from *Stevia rebaudiana.* Artificial sweeteners, sugar alcohol and extracted sweeteners all taste many times - some hundreds of times - sweeter than sugar, so that the amounts added needed to create the same level of sweetness are dramatically lower than sucrose.

*Stevia rebaudiana* is a crop of important economic value, and has a very high content of stevioside in the leaves thereof. At present, more than 100 compounds have been identified in *Stevia rebaudiana,* and the best known one is stevioside, and in particular, steviol glycoside and Rebaudioside A (J. Agric. Food Chem., 2012, 60, 886-895). Recently a novel stevioside has been found in a hybrid *Stevia rebaudiana* plant Morita (2010, J. Appl. Glycosci., 57, 199-209).

Patent Application No. 201310353500.9 discloses a biological method for preparing Rebaudioside M, where Rebaudioside A or Rebaudioside D is used as a substrate, and in the presence of a glucosyl donor, Rebaudioside M is generated by means of reaction of the substrate under the catalysis of UDP-glucosyl transferase and/or recombinant cells containing the UDP-glucosyl transferase. However, the process of the patent is not sufficiently optimized, with a conversion rate of only about 80% at most and a purity of only 95%, as well as a lower substrate concentration and a higher production cost. Thus, the process is not suitable for industrialized production.

### Summary

A technical issue to be solved in the present invention is to provide a method for preparing Rebaudioside M by an enzyme method in order to overcome drawbacks in the prior art. This method can produce a Rebaudioside M product with a higher purity at a lower cost.

In order to solve the above technical issue, the present invention employs the following technical solution:
A method for preparing Rebaudioside M by using an enzyme method, where Rebaudioside A or Rebaudioside D is used as a substrate, and in the presence of sucrose and UDP, Rebaudioside M is generated by means of reaction of the substrate under the catalysis of a mixture of UDP-glucosyl transferase and sucrose synthetase (AtSUS1), or recombinant cells containing the UDP-glucosyl transferase and sucrose synthetase. The reaction is carried out in an aqueous-phase system at a temperature of 20 to 60°C and pH 5.0 to 9.0. The reaction system further contains dimethyl sulfoxide at a concentration of 3% to 5% according to the ratio by volume for facilitating solubilization of the substrate. In the initial reaction system, the Rebaudioside A has a concentration greater than or equal to 10 g/L, and the Rebaudioside D has a concentration greater than or equal to 15 g/L. After the reaction, the reaction solution is centrifuged, then the supernatant is taken, and separated with a macroporous adsorbent resin to obtain a solution of a crude product of Rebaudioside M, which is crystallized in an aqueous ethanol solution, so as to obtain a Rebaudioside M product with a purity greater than 98%.

Preferably, the UDP-glucosyl transferase is UGT-A derived from *Stevia rebaudiana* and / or UGT-B derived from *Oryza sativa.*

Preferably, the reaction is carried out in an aqueous-phase system at a temperature of 35°C to 45°C and pH 6.5 to 8.5.

Preferably, the recombinant cells containing UDP-glucosyl transferase and sucrose synthetase are employed to carry out the catalysis, and the initial reaction system further contains toluene at a concentration of 1% to 3% according to the ratio by volume.

Preferably, a ratio by weight of the UDP-glucosyl transferase to the sucrose synthetase is 1:0.2 to 0.4.

Preferably, the method is implemented as follows: all the raw materials employed in the reaction are added into a reaction kettle, brought to a final volume with an aqueous-phase system, mixed uniformly, then placed at a set temperature, and stirred for reaction.

Preferably, the recombinant cells are microbial cells.

More preferably, the microorganism is *Escherichia coli, Saccharomyces cerevisiae* or *Pichia pastoris.*

Preferably, the substrate is Rebaudioside A, and the UDP-glucosyl transferase is a mixture of UGT-A derived from *Stevia rebaudiana* and UGT-B derived from *Oryza sativa,* where the amino acid sequence of UGT-A derived from *Stevia rebaudiana* is at least 80% consistent with Sequence 2, and the amino acid sequence of UGT-B derived from *Oryza sativa* is at least 80% consistent with Sequence 4. More preferably, in the mixture of UGT-A derived from *Stevia rebaudiana* and UGT-B derived from *Oryza sativa,* a ratio by weight of UGT-A derived from *Stevia rebaudiana* to UGT-B derived from *Oryza sativa* is 3 to 5:1.

Preferably, the substrate is Rebaudioside D, the UDP-glucosyl transferase is UGT-A derived from *Stevia rebaudiana,* and the amino acid sequence of UGT-A derived from *Stevia rebaudiana* is at least 80% consistent with Sequence 2.

Preferably, the reaction solution, after the reaction and before the centrifugation, is first heated at 50 to 60°C and subjected to ultrasonic treatment for 50 to 70 min.

Preferably, the solution of the crude product of Rebaudioside M is crystallized in the aqueous ethanol solution by particular steps as follows: the solution of the crude product of Rebaudioside M is concentrated by distillation at a reduced pressure and then centrifuged, and the supernatant is discarded; the precipitate is washed with added water and then centrifuged, and the supernatant is discarded; the precipitate is suspended with an aqueous ethanol solution at a concentration of 40% to 70% according to the ratio by volume, heated to 60-70°C for solubilization, and water is added therein until the aqueous ethanol solution has a concentration of 20 to 30% according to the ratio by volume; and the mixture is gradually cooled to room temperature, and subjected to crystallization and precipitation of a solid, followed by suction filtration and vacuum drying.

As a result of implementation of the above technical solutions, the present invention possesses the following advantages as compared with the prior art.

The method for preparing Rebaudioside M by an enzyme method provided in the present invention has an important application value. Through optimization and controlling of the solubilizer, reaction temperature and pH, the conversion rate and purity are both improved, and the production cost is reduced. Thus, this method is suitable for industrialized production.

### Brief Description of the Drawings

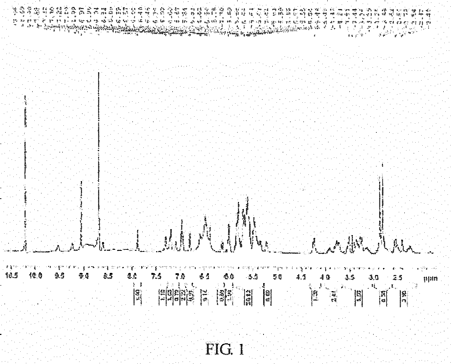

FIG. 1 is a proton magnetic spectrum diagram of a product obtained in Example 7 of the present invention.

### Detailed Description of the Preferred Embodiments

The present invention provides a process for synthesizing Rebaudioside M by using an enzyme method, with Rebaudioside A or Rebaudioside D as a raw material.

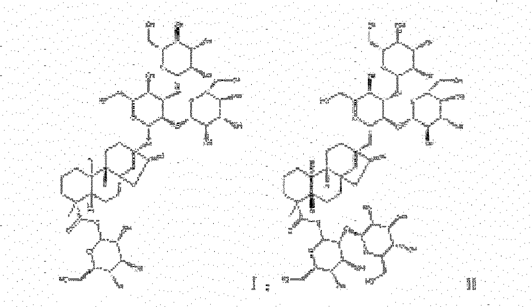

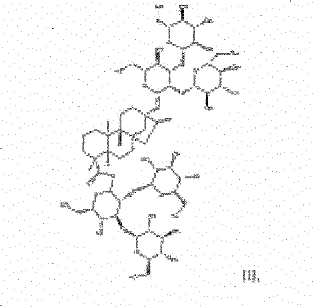

Rebaudioside A, Rebaudioside D and Rebaudioside M contain 4, 5 and 6 dextrose units of stevioside respectively, with structural formulae respectively seen in Formulae I, II and III.

The present invention provides two routes for synthesizing Rebaudioside M:
Route 1: 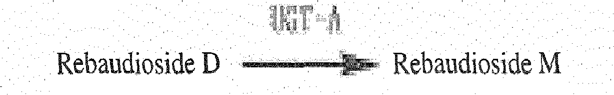 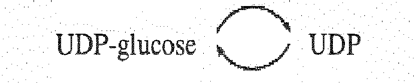 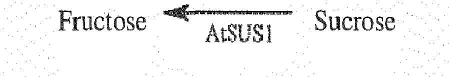
Route 2:

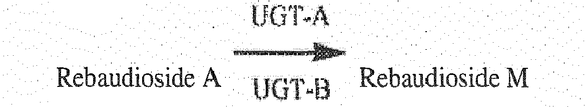

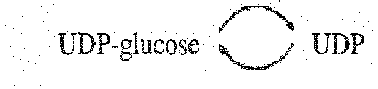

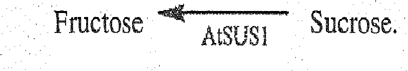

In the present invention, the raw material is purified.

According to one particular aspect of the present invention, the raw material comprises a steviol glycoside substrate Rebaudioside A.

According to another particular aspect of the present invention, the raw material comprises a steviol glycoside substrate Rebaudioside D.

According to the present invention, the UDP glucosyl transferase may be occurred in a form of a lyophilized enzyme powder that may or may not have been purified, or present in recombinant cells.

Recombinant cells containing UGT-A, UGT-B and AtSUS 1 (a sucrose synthetase) are obtained by the following method.

Recombinant *Escherichia coli* (or other microbial bacteria) expression strains of UGT-A, UGT-B and AtSUS 1 are obtained by utilizing molecular cloning techniques and genetic engineering techniques. Then, the recombinant *Escherichia coli* are fermented, and subjected to aftertreatment and collection of the recombinant cells.

The molecular cloning techniques and genetic engineering techniques described in the present invention are all known. Molecular cloning techniques may be seen in Molecular Cloing: A Laboratory Manual. 3rd Edition, by J. Shambrook, 2005.

Expression steps for constructing the recombinant strains of the present invention by employing the genetic engineering technique are as follows:
(1) gene fragments of UGT-A and AtSUS1 are subcloned respectively into two multiclonal sites MCS2 (NdeI/XhoI) and MCS1 (BamHI/HindIII) of pACYC-Duet-1, to obtain a plasmid pA-UGTA-SUS1;
(2) the UGT-B gene is subcloned into a site between the NdeI and BamHI sites of pET30a, to obtain a recombinant plasmid pE-UGIT-B; and
(3) PA-UGT-A-SLTS1 and pE-UGT-B are transformed successively into *Escherichia coli* BL21 (DE3), to obtain a strain GQ-ABS.

The recombinant cells containing UGT, or the lyophilized UGT powders are prepared by utilizing the recombinant *Escherichia coli* expression strains containing UGT, by steps as follows:
The recombinant *Escherichia coli* expression strain GQ-ABS is inoculated into 4 mL of a liquid LB medium according to a proportion of 1%, and cultured overnight with shaking (200 rpm) at 37°C. The culture that has experienced overnight culturing is transferred to 50 mL of the liquid LB medium in an inoculum size of 1%. The culture medium is cultured with shaking (200 rpm) at 37°C to an OD600 value up to 0.6-0.8. IPTG at a final concentration of 0.4 mM is added therein, and the mixture is cultured overnight with shaking at 20°C. After completion of the induction, cells are collected by centrifugation (8,000 rpm, 10 min). The cells are resuspended using 5 mL of a 2 mmol/L phosphate buffer (pH 7.0) to obtain the recombinant cells, or further ruptured ultrasonically in an ice bath, to obtain the lyophilized powder by centrifuging the ruptured liquid (8,000 rpm, 10 min), collecting the supernatant and lyophilizing for 24 h.

The present invention will be described below in more details in conjunction with particular examples.

### Example 1: preparation of recombinant Escherichia coli cells containing UGT-A

According to Sequence 1 and Sequence 2, UGT-A gene fragments were genetically synthesized, NdeI and BamHI enzyme cutting sites were added on both ends respectively, and pUC57 vectors (Suzhou Genewiz Biotech Co., Ltd.) were ligated therein. The UGT gene fragments were subjected to enzyme digestion with restriction endonucleases NdeI and BamHI. Purified fragments were recovered. T4 ligase was added therein, and the fragments were ligated into corresponding enzyme cutting sites of pET30a, to transform the BL21 (DE3) strains, so as to obtain a recombinant strain GQ-A.

The UGT strains were inoculated into 4 mL of a liquid LB medium according to a proportion of 1%, and cultured overnight with shaking (200 rpm) at 37°C. The culture that had experienced overnight culturing was transferred to 50 mL of the liquid LB medium in an inoculum size of 1%. The culture medium was cultured with shaking (200 rpm) at 37°C to an OD600 value up to 0.6-0.8. IPTG at a final concentration of 0.4 mM was added therein, and the mixture was cultured overnight with shaking at 20°C. After completion of the induction, cells were collected by centrifugation (8,000 rpm, 10 min). The cells were resuspended using 5 mL of a 2 mmol/L phosphate buffer (pH 7.0) to obtain recombinant cells containing UGT-A for use in the catalysis.

### Example 2: preparation of lyophilized UGT-A powder

The recombinant cells of UGT-A prepared in Example 1 were ruptured ultrasonically in an ice bath, to obtain a lyophilized powder of UGT-A by centrifuging the ruptured liquid (8,000 rpm, 10 min), collecting the supernatant and lyophilizing for 24 h.

### Example 3: preparation of recombinant Escherichia coli cells containing UGT-B

According to Sequence 3 and Sequence 4, UGT-B gene fragments were genetically synthesized, NdeI and BamHI enzyme cutting sites were added on both ends respectively, and pUC57 vectors (Suzhou Genewiz Biotech Co., Ltd.) were ligated therein. The UGT gene fragments were subjected to enzyme digestion with restriction endonucleases NdeI and BamHI. Purified fragments were recovered. T4 ligase was added therein, and the fragments were ligated into corresponding enzyme cutting sites of pET30a, to transform the BL21 (DE3) strains, so as to obtain a recombinant strain GQ-B.

The UGT strains were inoculated into 4 mL of a liquid LB medium according to a proportion of 1%, and cultured overnight with shaking (200 rpm) at 37°C. The culture that had experienced overnight culturing was transferred to 50 mL of the liquid LB medium in an inoculum size of 1%. The culture medium was cultured with shaking (200 rpm) at 37°C to an OD600 value up to 0.6-0.8. IPTG at a final concentration of 0.4 mM was added therein, and the mixture was cultured overnight with shaking at 20°C. After completion of the induction, cells were collected by centrifugation (8,000 rpm, 10 min). The cells were resuspended using 5 mL of a 2 mmol/L phosphate buffer (pH 7.0) to obtain recombinant cells containing UGT-B for use in the catalysis.

### Example 4: preparation of lyophilized UGT-B powder

The recombinant cells of UGT-B prepared in Example 3 were ruptured ultrasonically in an ice bath, to obtain a lyophilized powder of UGT-B by centrifuging the ruptured liquid (8,000 rpm, 10 min), collecting the supernatant and lyophilizing for 24 h.

### Example 5: preparation of recombinant Escherichia coli cells containing UGT-A and AtSUS1

Gene fragments of UGT-A and AtSUS1 were inserted respectively into NdeI/XhoI and BamHI/HindIII sites of the pACYC-Duet-1 plasmid, to obtain a plasmid pA-UGT-A-SUS1. The plasmid was transformed into BL21 (DE3), to obtain a recombinant strain GQ-AS.

The UGT strains were inoculated into 4 mL of a liquid LB medium according to a proportion of 1%, and cultured overnight with shaking (200 rpm) at 37°C. The culture that had experienced overnight culturing was transferred to 50 mL of the liquid LB medium in an inoculum size of 1%. The culture medium was cultured with shaking (200 rpm) at 37°C to an OD600 value up to 0.6-0.8. IPTG at a final concentration of 0.4 mM was added therein, and the mixture was cultured overnight with shaking at 20°C. After completion of the induction, cells were collected by centrifugation (8,000 rpm, 10 min). The cells were resuspended using 5 mL of a 2 mmol/L phosphate buffer (pH 7.0) to obtain recombinant cells containing UGT-A and AtSUS1 for use in the catalysis.

### Example 6: preparation of recombinant Escherichia coli cells containing UGT-A, UGT-B and AtSUS1

According to Sequence 3 and Sequence 4, UGT-B gene fragments were genetically synthesized, NdeI and BamHI enzyme cutting sites were added on both ends respectively, and pUC57 vectors (Suzhou Genewiz Biotech Co., Ltd.) were ligated therein. The UGT gene fragments were subjected to enzyme digestion with restriction endonucleases NdeI and BamHI. Purified fragments were recovered. T4 ligase was added therein, and the fragments were ligated into corresponding enzyme cutting sites of pET30a, to transform the GQ-AS strains, so as to obtain a recombinant strain GQ-ABS.

The UGT strains were inoculated into 4 mL of a liquid LB medium according to a proportion of 1%, and cultured overnight with shaking (200 rpm) at 37°C. The culture that had experienced overnight culturing was transferred to 50 mL of the liquid LB medium in an inoculum size of 1%. The culture medium was cultured with shaking (200 rpm) at 37°C to an OD600 value up to 0.6-0.8. IPTG at a final concentration of 0.4 mM was added therein, and the mixture was cultured overnight with shaking at 20°C. After completion of the induction, cells were collected by centrifugation (8,000 rpm, 10 min). The cells were resuspended using 5 mL of a 2 mmol/L phosphate buffer (pH 7.0) to obtain recombinant cells containing UGT-A, UGT-B and AtSUS1 for use in the catalysis.

### Example 7: synthesis of Rebaudioside M by an enzyme method with Rebaudioside D as a substrate

0.224 g of UDP, 34.2 g of sucrose, 1.6 g of Rebaudioside D, 1 g of lyophilized UGT-A powder, 0.4 g of lyophilized AtSUS powder, 4 mL of dimethyl sulfoxide and 0.05 mol/L phosphate buffer (pH 8.0) were added successively into the reaction system to a final volume of 100 mL, mixed uniformly, then placed in a water bath at 37°C, and stirred at 200 rpm to carry out reaction for 18 h. After completion of the reaction, 200 µl of the reaction solution was taken and added into 800 µl of anhydrous methanol and mixed uniformly. The mixture was centrifuged for 5 min at 10,000 rpm. The supernatant was taken and passed through a filter membrane, followed by detection using high performance liquid chromatography (chromatographic condition: chromatographic column: Agilent eclipse sb-C18 4.6 X 150 mm; detection wavelength: 210 nm; mobile phase: methanol: water = 68%: 32%; flow rate: 1.0 mL/min; column temperature: 30°C). A conversion rate of Rebaudioside D was more than 90%. After completion of the reaction, 300 mL of deionized water was added into 100 mL of the reaction solution, and the mixture was heated for 1 h at 55°C, subjected to ultrasonic treatment, and centrifuged for 30 min at 6,700 rpm. The supernatant was a sample A. After the centrifugation, 100 mL of water was added into the precipitate, and the mixture was heated for 0.5 h at 55°C, subjected to ultrasonic treatment, and centrifuged for 30 min at 6,700 rpm. The supernatant was a sample B. The sample A and the sample B were mixed to then obtain a sample C, which was separated with a macroporous adsorbent resin (AB-8). The resultant was first flushed with 4 column volumes of water, and then eluted with 3.5 column volumes of 70% ethanol, to obtain a solution of the crude product of Rebaudioside M. The above solution of the crude product was distilled at a reduced pressure (40 to 50°C) until the remaining solution was about 10 mL, and centrifuged for 10 min at 9,900 rpm, and the supernatant was discarded. The precipitate was washed with 4 mL of water added, centrifuged for 10 min at 9,900 rpm, and the supernatant was discarded. The precipitate was suspended with a 50% aqueous ethanol solution, and heated to 65°C for solubilization. An equal volume of water was added therein, to an ethanol concentration of 25%. The mixture was gradually cooled to room temperature, and subjected to suction filtration and vacuum drying after precipitation of a solid, to obtain 1.12 g of Rebaudioside M, with a purity greater than 99%.

### Example 8: synthesis of Rebaudioside M by an enzyme method with Rebaudioside A as a substrate

0.18 g of UDP, 41.04 g of sucrose, 1 g of Rebaudioside A, 2 g of lyophilized UGT-A powder, 0.5 g of lyophilized UGT-B powder, 0.5 g of lyophilized AtSUS1 powder, 4 mL of dimethyl sulfoxide and 0.05 mol/L phosphate buffer (pH 7.0) were added successively into the reaction system to a final volume of 100 mL, mixed uniformly, then placed in a water bath at 37°C, and stirred at 200 rpm to carry out reaction for 18 h. After completion of the reaction, 200 µl of the reaction solution was taken and added into 800 µl of anhydrous methanol and mixed uniformly. The mixture was centrifuged for 5 min at 10,000 rpm. The supernatant was taken and passed through a filter membrane, followed by detection using high performance liquid chromatography (chromatographic condition: chromatographic column: Agilent eclipse sb-C18 4.6 X 150 mm; detection wavelength: 210 nm; mobile phase: methanol: water = 68%: 32%; flow rate: 1.0 mL/min; column temperature: 30°C). A conversion rate of Rebaudioside A was more than 90%. After completion of the reaction, 300 mL of deionized water was added into 100 mL of the reaction solution, and the mixture was heated for 1 h at 55°C, subjected to ultrasonic treatment, and centrifuged for 30 min at 6,700 rpm. The supernatant was a sample A. After the centrifugation, 100 mL of water was added into the precipitate, and the mixture was heated for 0.5 h at 55°C, subjected to ultrasonic treatment, and centrifuged for 30 min at 6,700 rpm. The supernatant was a sample B. The sample A and the sample B were mixed to then obtain a sample C, which was separated with a macroporous adsorbent resin (AB-8). The resultant was first flushed with 4 column volumes of water, and then eluted with 3.5 column volumes of 70% ethanol, to obtain a solution of the crude product of Rebaudioside M. The above solution of the crude product was distilled at a reduced pressure (40 to 50°C) until the remaining solution was about 10 mL, and centrifuged for 10 min at 9,900 rpm, and the supernatant was discarded. The precipitate was washed with 4 mL of water added, centrifuged for 10 min at 9,900 rpm, and the supernatant was discarded. The precipitate was suspended with a 50% aqueous ethanol solution, and heated to 65°C for solubilization. An equal volume of water was added therein, to an ethanol concentration of 25%. The mixture was gradually cooled to room temperature, and subjected to suction filtration and vacuum drying after precipitation of a solid, to obtain 0.69 g of Rebaudioside M, with a purity greater than 99%.

### Example 9: synthesis of Rebaudioside M by whole cell synthesis with Rebaudioside D as a substrate

The GQ-AS recombinant cell employed in the example was a recombinant strain containing both UGT-A and AtSUS 1.

0.045 g of UDP, 10.26 g of sucrose, 2 mL of toluene, 0.2 g of Rebaudioside D, 10 g of recombinant cells containing GQ-AS, 4 mL of dimethyl sulfoxide and 0.05 mol/L phosphate buffer (pH 8.0) were added successively into the reaction system to a final volume of 100 mL, mixed uniformly, then placed in a water bath at 37°C, and stirred at 200 rpm to carry out reaction for 7 h. After completion of the reaction, 200 µl of the reaction solution was taken and added into 800 µl of anhydrous methanol and mixed uniformly. The mixture was centrifuged for 5 min at 10,000 rpm. The supernatant was taken and passed through a filter membrane, followed by detection using high performance liquid chromatography (chromatographic condition: chromatographic column: Agilent eclipse sb-C18 4.6 X 150 mm; detection wavelength: 210 nm; mobile phase: methanol: water = 68%: 32%; flow rate: 1.0 mL/min; column temperature: 30°C). A conversion rate of Rebaudioside D was more than 90%. After completion of the reaction, 300 mL of deionized water was added into 100 mL of the reaction solution, and the mixture was heated for 1 h at 55°C, subjected to ultrasonic treatment, and centrifuged for 30 min at 6,700 rpm. The supernatant was a sample A. After the centrifugation, 100 mL of water was added into the precipitate, and the mixture was heated for 0.5 h at 55°C, subjected to ultrasonic treatment, and centrifuged for 30 min at 6,700 rpm. The supernatant was a sample B. The sample A and the sample B were mixed to then obtain a sample C, which was separated with a macroporous adsorbent resin (AB-8). The resultant was first flushed with 4 column volumes of water, and then eluted with 3.5 column volumes of 70% ethanol, to obtain a solution of the crude product of Rebaudioside M. The above solution of the crude product was distilled at a reduced pressure (40 to 50°C) until the remaining solution was about 10 mL, and centrifuged for 10 min at 9,900 rpm, and the supernatant was discarded. The precipitate was washed with 4 mL of water added, centrifuged for 10 min at 9,900 rpm, and the supernatant was discarded. The precipitate was suspended with a 50% aqueous ethanol solution, and heated to 65°C for solubilization. An equal volume of water was added therein, to an ethanol concentration of 25%. The mixture was gradually cooled to room temperature, and subjected to suction filtration and vacuum drying after precipitation of a solid, to obtain 0.14 g of Rebaudioside M, with a purity greater than 99%.

### Example 10: synthesis of Rebaudioside M by whole cell synthesis with Rebaudioside A as a substrate

The GQ-ABS recombinant cell employed in the example was a recombinant strain containing UGT-A, UGT-B and AtSUS1 at the same time.

0.045 g of UDP, 10.26 g of sucrose, 2 mL of toluene, 0.2 g of Rebaudioside A, 10 g of recombinant cells also containing GQ-ABS, 4 mL of dimethyl sulfoxide and 0.05 mol/L phosphate buffer (pH 7.0) were added successively into the reaction system to a final volume of 100 mL, mixed uniformly, then placed in a water bath at 37°C, and stirred at 200 rpm to carry out reaction for 7 h. After completion of the reaction, 200 µl of the reaction solution was taken and added into 800 µl of anhydrous methanol and mixed uniformly. The mixture was centrifuged for 5 min at 10,000 rpm. The supernatant was taken and passed through a filter membrane, followed by detection using high performance liquid chromatography (chromatographic condition: chromatographic column: Agilent eclipse sb-C18 4.6 X 150 mm; detection wavelength: 210 nm; mobile phase: methanol: water = 68%: 32%; flow rate: 1.0 mL/min; column temperature: 30°C). A conversion rate of Rebaudioside A was more than 40%. After completion of the reaction, 300 mL of deionized water was added into 100 mL of the reaction solution, and the mixture was heated for 1 h at 55°C, subjected to ultrasonic treatment, and centrifuged for 30 min at 6,700 rpm. The supernatant was a sample A. After the centrifugation, 100 mL of water was added into the precipitate, and the mixture was heated for 0.5 h at 55°C, subjected to ultrasonic treatment, and centrifuged for 30 min at 6,700 rpm. The supernatant was a sample B. The sample A and the sample B were mixed to then obtain a sample C, which was separated with a macroporous adsorbent resin (AB-8). The resultant was first flushed with 4 column volumes of water, and then eluted with 3.5 column volumes of 70% ethanol, to obtain a solution of the crude product of Rebaudioside M. The above solution of the crude product was distilled at a reduced pressure (40 to 50°C) until the remaining solution was about 10 mL, and centrifuged for 10 min at 9,900 rpm, and the supernatant was discarded. The precipitate was washed with 4 mL of water added, centrifuged for 10 min at 9,900 rpm, and the supernatant was discarded. The precipitate was suspended with a 50% aqueous ethanol solution, and heated to 65°C for solubilization. An equal volume of water was added therein, to an ethanol concentration of 25%. The mixture was gradually cooled to room temperature, and subjected to suction filtration and vacuum drying after precipitation of a solid, to obtain 0.05 g of Rebaudioside M, with a purity greater than 99%.

The above examples are only intended for the description of technical conception and feature of the present invention, for the purpose of enabling those familiar with the art to understand and thereby implement the contents of the present invention, rather than limiting the protection scope of the present invention therewith. Any equivalent changes or modifications made according to the spirit and essence of the present invention shall be encompassed within the protection scope of the present invention.

### ADDITIONAL DISCLOSURE

The following clauses are offered as further description of the disclosed invention.
1. A method for preparing Rebaudioside M by using an enzyme method, wherein Rebaudioside A or Rebaudioside D is used as a substrate, and in the presence of sucrose and UDP, Rebaudioside M is generated by means of reaction of the substrate under the catalysis of a mixture of UDP-glucosyl transferase and sucrose synthetase, or recombinant cells containing the UDP-glucosyl transferase and sucrose synthetase, the reaction is carried out in an aqueous-phase system at a temperature of 20 to 60°C and pH 5.0 to 9.0, wherein, in the initial reaction system, the Rebaudioside A has a concentration greater than or equal to 10 g/L, the Rebaudioside D has a concentration greater than or equal to 15 g/L, and after the reaction, the reaction solution is centrifuged, then the supernatant is separated with a macroporous adsorbent resin to obtain a solution of a crude product of Rebaudioside M, which is crystallized in an aqueous ethanol solution, so as to obtain the Rebaudioside M product with a purity greater than 98%.
2. The method according to Clause 1, wherein, in the initial reaction system, the Rebaudioside A has a concentration of 10 to 30 g/L, and the Rebaudioside D has a concentration of 15 to 50 g/L.
3. The method according to Clause 1, wherein, the UDP-glucosyl transferase is UGT-A derived from *Stevia rebaudiana* and/or UGT-B derived from *Oryza sativa.*
4. The method according to Clause 1, wherein, the reaction is carried out in an aqueous-phase system at a temperature of 35°C to 45°C and pH 6.5 to 8.5.
5. The method according to Clause 1, wherein, the recombinant cells containing the UDP-glucosyl transferase and sucrose synthetase are employed in the reaction to carry out the catalysis, and the initial reaction system further contains toluene or other cellular permeating agents at a concentration 1% to 3% according to the ratio by volume.
6. The method according to Clause 1, wherein, a ratio by weight of the UDP-glucosyl transferase to the sucrose synthetase is 1:0.2 to 0. 4.
7. The method according to Clause 1, wherein, the method is implemented as follows: all the raw materials employed in the reaction are added into a reaction kettle, brought to a final volume with an aqueous-phase system, mixed uniformly, then placed at a set temperature, and stirred for reaction.
8. The method according to Clause 1, wherein, the recombinant cells are microbial cells.
9. The method according to Clause 8, wherein, the microorganism is *Escherichia coli, Saccharomyces cerevisiae* or *Pichia pastoris.*
10. The method according to any one of Clauses 1 to 9, wherein, the substrate is Rebaudioside A, the UDP-glucosyl transferase is a mixture of UGT-A derived from *Stevia rebaudiana* and UGT-B derived from *Oryza sativa,* wherein the amino acid sequence of UGT-A derived from *Stevia rebaudiana* is at least 80% consistent with Sequence 2, and the amino acid sequence of UGT-B derived from *Oryza sativa* is at least 80% consistent with Sequence 4.
11. The method according to Clause 10, wherein, in the mixture of UGT-A derived from *Stevia rebaudiana* and UGT-B derived from *Oryza sativa,* a ratio by weight of UGT-A derived from *Stevia rebaudiana* to UGT-B derived from *Oryza sativa* is 3 to 5:1.
12. The method according to any one of Clauses 1 to 9, wherein, the substrate is Rebaudioside D, the UDP-glucosyl transferase is UGT-A derived from *Stevia rebaudiana,* and the amino acid sequence of UGT-A derived from *Stevia rebaudiana* is at least 80% consistent with Sequence 2.
13. The method according to Clause 1, wherein, the reaction solution, after the reaction and before the centrifugation, is first heated at 50 to 60°C and subjected to ultrasonic treatment for 50 to 70 min.
14. The method according to Clause 1, wherein, the solution of the crude product of Rebaudioside M is crystallized in the aqueous ethanol solution by the particular steps as follows: the solution of the crude product of Rebaudioside M is concentrated by distillation at a reduced pressure and then centrifuged, and the supernatant is discarded; the precipitate is washed with added water and then centrifuged, and the supernatant is discarded; the precipitate is suspended with an aqueous ethanol solution at a concentration of 40% to 70% according to the ratio by volume, heated to 60-70°C for solubilization, and water is added therein until the aqueous ethanol solution has a concentration of 20 to 30% according to the ratio by volume; and the mixture is gradually cooled to room temperature, and subjected to crystallization and precipitation of a solid, followed by suction filtration and vacuum drying.

## Claims

1. A method for preparing rebaudioside M with a purity greater than 98%, the method comprising
(i) reacting rebaudioside D, in an aqueous phase reaction solution, with a glucosyl donor in the presence of recombinant cells, each of the recombinant cells comprising one or more exogenous polynucleotides encoding:
a) a UDP-glucosyl transferase having an amino acid sequence with at least 80% identity to SEQ ID NO: 2; and
b) a sucrose synthetase;
(ii) isolating crude rebaudioside M; and
(iii) crystalizing the crude rebaudioside M to obtain rebaudioside M with a purity greater than 98%.

2. The method of claim 1, wherein the rebaudioside D is prepared *in situ* by reacting rebaudioside A, in the aqueous phase reaction solution, with the glucosyl donor in the presence of the recombinant cells, wherein each of the recombinant cells further comprises an exogenous polynucleotide encoding a UDP-glucosyl transferase having an amino acid sequence with at least 80% identity to SEQ ID NO: 4.

3. The method of claim 1 or claim 2, wherein in the aqueous phase reaction solution, the rebaudioside D has a concentration greater than or equal to 15 g/L.

4. The method of claim 3, wherein in the aqueous phase reaction solution, the rebaudioside D has a concentration of 15 to 50g/L.

5. The method of any one of claims 1 to 4, wherein the aqueous phase reaction solution comprises a solubilizer for the rebaudioside D.

6. The method of claim 5, wherein the solubilizer comprises dimethyl sulfoxide at a concentration of 3% to 5% by volume.

7. The method of any foregoing claim, wherein the glucosyl donor is UDP-glucose generated *in situ* from UDP and sucrose in the presence of the sucrose synthetase.

8. The method of any foregoing claim, wherein the recombinant cells are microbial cells selected from the group consisting of: *Escherichia coli, Saccharomyces cerevisiae,* and *Pichia pastoris.*

9. The method of any foregoing claim, wherein the reaction solution further comprises a phosphate buffer.

10. The method of any foregoing claim, wherein the reaction solution has a temperature ranging from 35°C to 45°C.

11. The method of any foregoing claim, wherein the reaction solution has a pH ranging from 6.5 to 8.5.

12. The method of any foregoing claim, wherein the reaction solution further comprises toluene or other cellular permeating agent at a concentration of 1% to 3% by volume.


SEQUENCE LISTING

<110>  PEPSICO, INC.
       TAO, JUNHUA
       LI, GUOQING
       LIANG, XIAOLIANG
       LEE, THOMAS
       YEP, GREGORY
       HOU, MAOQI
       TAO, ANDREW

<120>  REBAUDIOSIDE M ENZYMATIC PREPARATION METHOD

<130>  3711.819PC00/JMC/MSB

<140>  PCT/CN2014/071715
<141>  2014-01-28

<160>  4

<170>  PatentIn version 3.5

<210>  1
<211>  1377
<212>  DNA
<213>  Artificial Sequence

<220>
<223>  Synthesized nucleotide sequence

<400>  1
atggaaaaca aaaccgaaac cacggtacgc cgtcgtcgtc gtatcatcct cttcccggtt      60

ccgtttcagg gtcacatcaa cccgatcctt cagttggcaa acgtactgta ctctaaaggt     120

tttagcatca ccatttttca cactaacttt aacaaaccga aaacctctaa ctatccgcac     180

ttcactttcc gcttcatcct ggacaacgac ccgcaagatg agcgcattag caacctgccg     240

acccatggcc cgctggcagg catgcgcatc cctatcatca atgaacacgg cgctgacgaa     300

ctgcgtcgtg agctggaact cctgatgctg gcttctgaag aagacgagga agtgtcttgc     360

ctgattacag acgctctctg gtactttgct cagagcgtgg cggactctct gaacctgcgc     420

cgtctggttc ttatgacttc ttccttgttt aatttccatg cgcatgtctc tctgccgcag     480

ttcgacgagc tgggctacct ggacccggat gacaaaactc gcctggagga acaggcatct     540

ggcttcccga tgctgaaagt aaaagatatc aaaagcgcat actccaattg gcagatcctg     600

aaagagattc tgggcaaaat gatcaagcag actaaagcat ccagcggcgt tatctggaac     660

tcctttaaag agctggagga aagcgaactg gaaaccgtga tccgtgaaat cccggcaccg     720

tcgttcctga ttcctctgcc taaacatctg accgcctcct cttcttctct gctggatcac     780

gatcgcaccg ttttccagtg gctggatcag caaccgccga gttctgtgct gtatgtttct     840

ttcggctcga cgagtgaggt tgacgaaaaa gacttcctgg aaatcgcacg cggcctggtt     900

gactctaaac agagctttct gtgggttgta cgtccgggtt tcgtgaaggg cagcacctgg     960

gttgaaccgc tgccggacgg ctttttgggc gaacgcggcc gtatcgtaaa atgggtaccg 1020

cagcaggagg tactggcaca cggcgcaatt ggggcgttct ggactcactc cggctggaac 1080

tccactctgg aatccgtatg cgaaggcgtt cctatgattt tcagcgactt cggcctggat 1140

cagccgctga acgcacgcta tatgtcagac gttctgaaag tcggtgtgta tctggagaac 1200

gggtgggagc gtggcgaaat tgccaacgcg atccgtcgtg ttatggtgga tgaagaaggc 1260

gaatacatcc gtcagaacgc tcgtgtcctt aaacagaaag ctgacgtgag cctgatgaaa 1320

ggtggctcta gctacgaatc gctggagtcc ctggtttctt acatctcgtc gctgtaa 1377

<210> 2
<211> 458
<212> PRT
<213> Artificial Sequence

<220>
<223> Synthesized protein sequence

<400> 2

Met Glu Asn Lys Thr Glu Thr Thr Val Arg Arg Arg Arg Arg Ile Ile
1               5                   10                  15

Leu Phe Pro Val Pro Phe Gln Gly His Ile Asn Pro Ile Leu Gln Leu
            20                  25                  30

Ala Asn Val Leu Tyr Ser Lys Gly Phe Ser Ile Thr Ile Phe His Thr
        35                  40                  45

Asn Phe Asn Lys Pro Lys Thr Ser Asn Tyr Pro His Phe Thr Phe Arg
    50                  55                  60

Phe Ile Leu Asp Asn Asp Pro Gln Asp Glu Arg Ile Ser Asn Leu Pro
65                  70                  75                  80

Thr His Gly Pro Leu Ala Gly Met Arg Ile Pro Ile Ile Asn Glu His
                85                  90                  95

Gly Ala Asp Glu Leu Arg Arg Glu Leu Glu Leu Leu Met Leu Ala Ser
            100                 105                 110

Glu Glu Asp Glu Glu Val Ser Cys Leu Ile Thr Asp Ala Leu Trp Tyr
        115                 120                 125

Phe Ala Gln Ser Val Ala Asp Ser Leu Asn Leu Arg Arg Leu Val Leu
    130                 135                 140

Met Thr Ser Ser Leu Phe Asn Phe His Ala His Val Ser Leu Pro Gln
145                 150                 155                 160

Phe Asp Glu Leu Gly Tyr Leu Asp Pro Asp Asp Lys Thr Arg Leu Glu
                165                 170                 175

Glu Gln Ala Ser Gly Phe Pro Met Leu Lys Val Lys Asp Ile Lys Ser
            180                 185                 190

Ala Tyr Ser Asn Trp Gln Ile Leu Lys Glu Ile Leu Gly Lys Met Ile
        195                 200                 205

Lys Gln Thr Lys Ala Ser Ser Gly Val Ile Trp Asn Ser Phe Lys Glu
    210                 215                 220

Leu Glu Glu Ser Glu Leu Glu Thr Val Ile Arg Glu Ile Pro Ala Pro
225                 230                 235                 240

Ser Phe Leu Ile Pro Leu Pro Lys His Leu Thr Ala Ser Ser Ser Ser
                245                 250                 255

Leu Leu Asp His Asp Arg Thr Val Phe Gln Trp Leu Asp Gln Gln Pro
            260                 265                 270

Pro Ser Ser Val Leu Tyr Val Ser Phe Gly Ser Thr Ser Glu Val Asp
        275                 280                 285

Glu Lys Asp Phe Leu Glu Ile Ala Arg Gly Leu Val Asp Ser Lys Gln
    290                 295                 300

Ser Phe Leu Trp Val Val Arg Pro Gly Phe Val Lys Gly Ser Thr Trp
305                 310                 315                 320

Val Glu Pro Leu Pro Asp Gly Phe Leu Gly Glu Arg Gly Arg Ile Val
                325                 330                 335

Lys Trp Val Pro Gln Gln Glu Val Leu Ala His Gly Ala Ile Gly Ala
            340                 345                 350

Phe Trp Thr His Ser Gly Trp Asn Ser Thr Leu Glu Ser Val Cys Glu
        355                 360                 365

Gly Val Pro Met Ile Phe Ser Asp Phe Gly Leu Asp Gln Pro Leu Asn
    370                 375                 380

Ala Arg Tyr Met Ser Asp Val Leu Lys Val Gly Val Tyr Leu Glu Asn
385                 390                 395                 400

Gly Trp Glu Arg Gly Glu Ile Ala Asn Ala Ile Arg Arg Val Met Val

                405                 410                 415

Asp Glu Glu Gly Glu Tyr Ile Arg Gln Asn Ala Arg Val Leu Lys Gln
            420                 425                 430

Lys Ala Asp Val Ser Leu Met Lys Gly Gly Ser Ser Tyr Glu Ser Leu
        435                 440                 445

Glu Ser Leu Val Ser Tyr Ile Ser Ser Leu
    450                 455

<210> 3
<211> 1389
<212> DNA
<213> Artificial Sequence

<220>
<223> Synthesized nucleotide sequence

<400> 3
atggacagcg gttactcttc tagctatgct gcggcagccg gtatgcacgt agttatttgt 60

ccgtggctcg ctttcggtca cctcctgccg tgcctggacc tggcgcagcg cctggcatct 120

cgtggtcacc gtgtcagttt cgttagcacg ccgcgtaaca tctcacgtct gccgccggtc 180

cgtccggctc tggccccgct ggttgcgttc gttgcgctac ctctgccgcg cgttgaaggc 240

ttaccggatg gcgcagagtc taccaacgac gtgccgcacg atcgcccgga tatggttgaa 300

ctccaccgcc gtgcatttga cggtctggca gctccgttct ccgaatttct gggtaccgcg 360

tgtgccgact gggtcatcgt agacgtattc caccactggg cagctgcagc ggctttagaa 420

cacaaagtac cgtgcgcaat gatgctgctg ggctctgctc acatgatcgc gtctattgcc 480

gaccgtcgtc tggaacgtgc agagaccgaa tctccagcgg cagccggtca gggccgtcct 540

gcagctgctc cgaccttcga agttgctcgt atgaagctca tccgcactaa aggttcttcc 600

ggtatgtcac tggcagagcg tttctcgctg acgctctccc gtagcagcct ggttgtgggg 660

cgctcctgcg tggaattcga accggaaact gtgccgctac tgtctaccct gcgtggcaag 720

ccgatcactt ttctgggtct catgccgcca ctgcacgaag gtcgccgcga agacggtgaa 780

gatgctacgg ttcgttggtt ggacgcccag ccggctaaaa gcgtcgtgta cgtagctctg 840

ggcagtgaag ttccattggg tgtcgagaaa gtgcatgaac tggctttggg tctggagctg 900

gctggcaccc gtttcctctg ggcactgcgt aagccgactg gtgtgtctga tgctgacctt 960

ctgccggctg gtttcgaaga acgtacccgt ggtcgcggcg tagtggcaac ccgctgggta 1020

ccgcagatgt ccatcctggc acacgctgct gttggcgcgt ttcttaccca ctgcgggtgg 1080

aactctacaa tcgaaggcct gatgttcggc catcctctga ttatgctgcc aatcttcggt 1140

gatcagggtc cgaacgctcg tctgatcgaa gccaaaaacg ccggcttaca agtcgcacgc 1200

aacgacggcg atggttcttt cgatcgtgaa ggtgttgcgg cagctatccg tgcagtggct 1260

gtagaagaag agtcgagcaa agtgttccag gcaaaagcca aaaagctgca ggaaatcgtt 1320

gcggacatgg cgtgccacga acgttacatc gatggcttta tccagcagct gcgctcctac 1380

aaagattaa 1389

<210> 4
<211> 462
<212> PRT
<213> Artificial Sequence

<220>
<223> Synthesized protein sequence

<400> 4

Met Asp Ser Gly Tyr Ser Ser Ser Tyr Ala Ala Ala Ala Gly Met His
1               5                   10                  15

Val Val Ile Cys Pro Trp Leu Ala Phe Gly His Leu Leu Pro Cys Leu
            20                  25                  30

Asp Leu Ala Gln Arg Leu Ala Ser Arg Gly His Arg Val Ser Phe Val
        35                  40                  45

Ser Thr Pro Arg Asn Ile Ser Arg Leu Pro Pro Val Arg Pro Ala Leu
    50                  55                  60

Ala Pro Leu Val Ala Phe Val Ala Leu Pro Leu Pro Arg Val Glu Gly
65                  70                  75                  80

Leu Pro Asp Gly Ala Glu Ser Thr Asn Asp Val Pro His Asp Arg Pro
                85                  90                  95

Asp Met Val Glu Leu His Arg Arg Ala Phe Asp Gly Leu Ala Ala Pro
            100                 105                 110

Phe Ser Glu Phe Leu Gly Thr Ala Cys Ala Asp Trp Val Ile Val Asp
        115                 120                 125

Val Phe His His Trp Ala Ala Ala Ala Ala Leu Glu His Lys Val Pro
    130                 135                 140

Cys Ala Met Met Leu Leu Gly Ser Ala His Met Ile Ala Ser Ile Ala
145                 150                 155                 160

Asp Arg Arg Leu Glu Arg Ala Glu Thr Glu Ser Pro Ala Ala Ala Gly
                165                 170                 175

Gln Gly Arg Pro Ala Ala Ala Pro Thr Phe Glu Val Ala Arg Met Lys
            180                 185                 190

Leu Ile Arg Thr Lys Gly Ser Ser Gly Met Ser Leu Ala Glu Arg Phe
        195                 200                 205

Ser Leu Thr Leu Ser Arg Ser Ser Leu Val Val Gly Arg Ser Cys Val
    210                 215                 220

Glu Phe Glu Pro Glu Thr Val Pro Leu Leu Ser Thr Leu Arg Gly Lys
225                 230                 235                 240

Pro Ile Thr Phe Leu Gly Leu Met Pro Pro Leu His Glu Gly Arg Arg
                245                 250                 255

Glu Asp Gly Glu Asp Ala Thr Val Arg Trp Leu Asp Ala Gln Pro Ala
            260                 265                 270

Lys Ser Val Val Tyr Val Ala Leu Gly Ser Glu Val Pro Leu Gly Val
        275                 280                 285

Glu Lys Val His Glu Leu Ala Leu Gly Leu Glu Leu Ala Gly Thr Arg
    290                 295                 300

Phe Leu Trp Ala Leu Arg Lys Pro Thr Gly Val Ser Asp Ala Asp Leu
305                 310                 315                 320

Leu Pro Ala Gly Phe Glu Glu Arg Thr Arg Gly Arg Gly Val Val Ala
                325                 330                 335

Thr Arg Trp Val Pro Gln Met Ser Ile Leu Ala His Ala Ala Val Gly
            340                 345                 350

Ala Phe Leu Thr His Cys Gly Trp Asn Ser Thr Ile Glu Gly Leu Met
        355                 360                 365

Phe Gly His Pro Leu Ile Met Leu Pro Ile Phe Gly Asp Gln Gly Pro
    370                 375                 380

Asn Ala Arg Leu Ile Glu Ala Lys Asn Ala Gly Leu Gln Val Ala Arg
385                 390                 395                 400

Asn Asp Gly Asp Gly Ser Phe Asp Arg Glu Gly Val Ala Ala Ala Ile
                405                 410                 415

Arg Ala Val Ala Val Glu Glu Glu Ser Ser Lys Val Phe Gln Ala Lys
            420                 425                 430

Ala Lys Lys Leu Gln Glu Ile Val Ala Asp Met Ala Cys His Glu Arg
        435                 440                 445

Tyr Ile Asp Gly Phe Ile Gln Gln Leu Arg Ser Tyr Lys Asp
    450                 455                 460